# EUROPEAN PATENT SPECIFICATION

(11) **EP 1 942 967 B1**
(45) Date of publication and mention of the grant of the patent: **01.08.2012**
(21) Application number: 06806455.9
(22) Date of filing: 23.10.2006
(51) Int. Cl.: A61M 15/00, B05B 11/02, B05B 11/00, F04B 53/16

(54) **METHOD FOR METERING MEDICAMENTS**
VERFAHREN ZUR DOSIERUNG VON MEDIKAMENTEN
MÉTHODE POUR DOSER DES MÉDICAMENTS

(30) Priority: 03.11.2005 DE 102005052898
(43) Date of publication of application: 16.07.2008
(73) Proprietor: Boehringer Ingelheim Pharma GmbH & Co. KG, 55216 Ingelheim am Rhein (DE)
(72) Inventor: BOECK, Georg, 88471 Laupheim (DE); GESER, Johannes, 55218 Ingelheim am Rhein (DE); HAUSMANN, Matthias, 46459 Rees (DE); KOELBEL, Hans-Juergen, 53721 Siegburg (DE); EICHER, Joachim, 44227 Dortmund (DE); FEIERTAG, Christian, 45549 Sprockhoevel (DE)
(74) Representative: Häckel, Stefan
(86) International application number: PCT/EP2006/010177
(87) International publication number: WO 2007/051536

(56) References cited:
- EP-A- 1 426 662
- WO-A1-2005/080001
- COULLARD C.R. ET AL: "Matching problems in selective assembly operations" ANNALS OF OPERATIONS RESEARCH, vol. 76, 1998, pages 95-107, XP002419369

## Description

The present invention relates to a method of assembling a device for delivering a medicament, particularly in the form of an aerosol, with improved metering accuracy and to a device for administering a medicament, particularly in the form of an aerosol, with improved metering accuracy, according to the preamble of claim 8.

By the term "medicaments" are also meant, according to the invention, in particular, medicament formulations or medicament mixtures. Preferably the medicament is in liquid form, and may be a suspension, a solution or a mixture of the two (a so-called suslution). It may also be a powder. The following description of the invention is directed primarily to a medicament in liquid form, and consequently it is often referred to just as a liquid. However, it also applies accordingly to other medicaments according to the present invention and comparable substances.

EP 1 426 662 A1, which constitutes the starting point for the present invention, discloses a device for pumping and preferably delivering a liquid, particularly a pharmaceutical liquid. The known device has a guide tube with a piston which is longitudinally guidable therein along its entire length, as well as an O-ring gasket for providing a seal between the guide tube and the piston. The O-ring gasket is arranged in a groove of the guide tube. To obtain a good seal, the O-ring should fill the groove to more than 90%. In practice, it has been found that the tolerances of the individual components lead to an inadequate seal, particularly against air, and hence may result in insufficient metering accuracy. Accurate metering is, however, essential, precisely when administering drugs or the like, as in the present invention.

The article "Matching problems in selective assembly operations" of C. R. Coullard et. al., Annals of Operations Research, vol. 76, 1998, pages 95-107, describes an optimized greedy algorithm wherein weighted matchings are proposed.

Object of the present invention is to provide a method of assembling a device with improved metering accuracy for delivering and administering medicaments, particularly in aerosol form.

The above object is achieved by a method according to claim 1. Advantageous embodiments are the subject of the subclaims.

According to the invention, in the process, a first and a second component are used for the delivery, the first component being produced in batches, with at least one significant magnitude of the first components of each batch being only randomly determined and from this at least one decisive significant magnitude for all the first components of the respective batch is determined, while the second component is divided into groups which differ by at least one essential magnitude of the second components, while as a function of the at least one decisive significant magnitude a matching (suitable) group is selected, while a first component of a batch is combined preferably exclusively with a second component of a group matching to this batch or is incorporated therein. By selecting a corresponding, matching group of second components, it is possible to achieve an improved seal between the combined components which are preferably moved relative to one another in order to deliver the medicament. In this way, improved metering accuracy is obtained.

The method is suitable particularly for very small components produced, for example, by microengineering or having dimensions of only some 10 µm to about 3 mm. For example, the first components are injection-moulded and form shaped seals, preferably annular-shaped, especially O-rings.

As significant magnitudes of the first components, particularly in the case of annular shaped seals such as O-rings, the volume and/or compressibility are preferably determined.

It has been found that it is sufficient to determine the average value and the standard deviation, e.g. of the volume and compressibility, as significant magnitudes of the first components. This allows relatively little effort.

The second components preferably comprise a recess, particularly a shoulder or groove, for accommodating the first component and form, in particular, a guide tube for a piston of the device. Preferably, an essential magnitude for dividing the second components into the groups is a magnitude relating to the recess, such as the depth and/or width of the recess. It has been found that these magnitudes or dimensions are sufficient for the division process, so that only relatively little effort is required.

Preferably the mean and the standard deviation, particularly the depth and/or width of the recess, are again used as essential magnitudes for division into the groups.

The second components are preferably deliberately manufactured with different essential magnitudes, these magnitudes differing by more than the manufacturing tolerance, so as to enable different groups of the second components to be produced and held in readiness. Manufacturing with different essential magnitudes is preferably carried out depending on the requirements or the statistical probability.

The second components are preferably also produced in batches, but particularly with different essential magnitudes, the essential magnitude of the second components being only randomly determined in each batch and from this the essential magnitude for all the second components in the respective batch being determined. Thus, individual measurement of the second components can be avoided and in this way the effort can be kept low overall.

Particularly preferably the device comprises, in addition to the first and second components, at least one further component, particularly a number of further components, such as a piston which is sealed off by the first component, and a support ring for axially securing the component on the second component. If different batches of the first and other components are then combined, a desired target value can be achieved by selecting a matching group of the second component which is consequently "variable" at least in its essential magnitude. For this variation, a measurement of the depth of the recess, i.e. the guide tube, and/or the width (axial length) of the recess, i.e. for example a support ring for securing a seal as the first component in the recess, is particularly suitable.

To allow the matching group to be selected when the device has at least one other component in addition to the first and second components, one or more other significant magnitude(s) of the other component or components, particularly the diameter of the piston and/or the axially effective length of the support ring, is or are determined and taken into account as one or more further significant magnitude(s) in addition to the decisive significant magnitude when selecting the fitting group.

As already explained, the matching group is selected so that the decisive significant magnitude together with optionally other significant magnitudes and the essential magnitude in the finished device leads - at least on average - to a specific desired value, particularly a desired fill level of the recess for a shaped seal. The selection is made, in particular, with computer assistance taking account of error propagation or statistical methods.

By the term "fill level" is meant, in the present invention, in particular, the quotient of the volume of the installed seal by the volume of the recess.

By the term "shaped seal" are meant in the present invention both flat seals and O-ring seals and other shaped seals, i.e. with different cross-sectional shapes. Preferably the shaped seals are constructed as a continuous ring.

The proposed process has been described above in general terms but with reference to its preferred use with a device for pumping or metering and preferably expelling a liquid or a medicament. The proposed process is generally applicable to all kinds of devices. The preferred use is with devices made up of micro-components, the individual dimensioning or measuring of such components would represent excessive effort or would be totally impossible. The description that follows is directed primarily to a proposed device.

A device assembled according to the method of the invention, for administering a medicament, particularly for pumping or metering and preferably expelling a liquid, has a guide tube with a piston which is longitudinally movable therein, a shaped seal for forming a seal between the guide tube and the piston and a recess for accommodating the shaped seal, the shaped seal of a specific batch of shaped seals being combined with a guide tube of a matching specific group, the group being selected from several groups of guide tubes, depending on at least one decisive significant magnitude of the batch, in order to fill the recess with the shaped seal to a desired fill level. Thus, a specific desired fill level which is provided by the desired seal and hence improved metering accuracy can be achieved in a relatively simple manner.

When choosing the matching group, the tolerances or magnitudes of other components, particularly significant magnitudes of batches of further components, such as the diameter of the piston, the effective axial length of the support ring for axially supporting the shaped seal or the boundaries of the groove or the like, may also be taken into consideration.

Other aspects, characteristics, advantages and features of the present invention will become apparent from the claims and the following description of a preferred embodiment with reference to the drawing. The single Figure shows a schematic section through a detail of a proposed device.

The proposed device 1 for administering or delivering, more particularly for pumping or metering, a medicament, preferably a liquid 2, is designed especially for very small pump volumes for metered amounts. The pump volume in the embodiment shown is from 1 µl to 1 ml, preferably from 1 µl to 500 µl, more particularly 5 µl to 100 µl, most preferably 5 µl to 30 µl, and in particular about 15 µl, per piston stroke.

In order to be able to ensure precise conveying and metering of the desired volume, in particular even on first actuation after a lengthy period of non-use, it is important that no air is able to get into the device 1, particularly while it is not being used, as otherwise the metering can no longer be carried out with the desired accuracy.

The device 1 has a guide tube 3 (second component), a piston 4 which is guided to be longitudinally movable therein (further component) and a shaped seal 5 (first component) in a recess 6 and optionally a support ring 7 (further component) for supporting the shaped seal 5.

The guide tube 3 forms optionally, together with the support ring 7, the recess 5 which annularly surrounds the piston 4 and is designed in particular as a groove, in this case as an annular groove. In the embodiment shown, the support ring 4 forms an axial side or boundary of the recess 5, so that the guide tube 3 essentially forms an annular shoulder and a radial outer boundary for the recess 5.

If required, the recess 5 may also be constructed separately from the guide tube 3.

In the embodiment shown the piston 4 has a circular cross-section with a diameter of from 0.25 mm to 4 mm, preferably 0.5 m to 3 mm, more particularly 0.75 mm to 2.25 mm.

The piston 4 is preferably made of metal, particularly stainless steel. It is constructed in particular as a hollow tube or capillary.

The piston 4 is preferably drawn and accordingly has a relatively small tolerance in terms of its diameter.

The shaped seal 5 is preferably of a continuous annular shape to correspond to the recess 6. In particular, the shaped seal 5 is an O-ring with an at least substantially circular cross-section in the uninstalled state.

The cross-section or material thickness of the uninstalled shaped seal 5 is from 0.3 mm to 3 mm, preferably 0.5 mm to 2 mm, particularly 1 mm to 1.5 mm, in the embodiment shown. The internal diameter corresponds substantially to the diameter of the piston.

The shaped seal 5 preferably consists of silicon or some other rubber-elastic material which is suitable in particular for use in the pharmaceutical or food industry.

In the installed state - i.e. when the device 1 is assembled - the shaped seal 5 is at least substantially accommodated in the recess 6. The support ring 7 bears axially thereon and fixes the shaped seal 5 axially in the recess 6. In addition, the shaped seal 5 bears radially against the piston 4 passing through the shaped seal 5, in a close fitting manner. The shaped seal 5 is pressed or deformed into the recess 6. Departing from the uninstalled state, the shaped seal 5 is substantially rectangular in cross-section or has at least one flat contact side for abutment on the piston 4.

The "fill level" corresponds to the quotient of the volume of the installed shaped seal 5 by the volume of the recess 6. In order to be able to obtain a good seal and correspondingly accurate metering with the device 1, the desired fill level, i.e. the "target fill level" is on average preferably 90%, more particularly at least 95%, with a tolerance of at most 5%, more particularly 4% or less.

In the embodiment shown, the support ring 7 is preferably attached to the delivery tube 3 by a cap-like retaining element 8 or the like. By corresponding axial or end-face contact surfaces it is possible to achieve a defined position of the support ring 7 and hence a defined width B (axial length) of the recess 6 for the shaped seal 5.

Moreover, the volume of the recess 6 is decisively determined by the depth T of the recess 6 in the delivery tube 3, i.e. the radial extent of the recess 6.

The piston 4 delimits a pump chamber 9 in the delivery tube 3. The piston 4 is preferably provided with a non-return valve 10 which is mounted in particular at the end of the piston 4 facing the pump chamber 9.

The preferably hollow piston 4 forms a supply channel 11 for the liquid 2 in the embodiment shown. The liquid 2 can be conveyed, more particularly sukked in, through the supply channel 11 via the inlet or non-return valve 10 into the pump chamber 9, with a suitable axial movement.

On the compression or delivery side the device 1 optionally has an outlet valve (not shown) and, for example, a nozzle 12 for delivering and optionally atomising the liquid 2.

The shaped seals 5 are produced in batches, i.e. groupwise. In particular, a batch is produced from a specified quantity of starting material which is as homogeneous as possible.

The shaped seals 5 are preferably produced by injection moulding, particularly by means of an injection moulding tool (not shown) having a plurality of cavities. Accordingly, in each injection moulding process, a plurality of shaped seals 5 is produced.

The shaped seals 5 may vary from one batch to the next, especially in terms of their significant magnitudes such as the diameter of the ring, cross-section, volume, compressibility or the like. Apart from the dimensions which are dependent on the tool (diameter of the ring, thickness, volume) variables which are determined by the material or the technicalities of the process, such as the compressibility, may vary.

The shaped seals 5 are first components according to the method proposed. The significant magnitudes (in particular the volume and compressibility only) of the shaped seals 5 are preferably determined only for some of all the shaped seals 5 in a batch and from them decisive significant magnitudes, particularly a mean and standard deviation, are determined, taking account of the various influences of tool-related dimensions and tolerances together with other dimensions, if applicable, and taking account of distribution functions.

The delivery tube 3 is classified according to the proposal by preferably only one essential magnitude, i.e. in this embodiment based on the depth T of the recess 6. The delivery tubes 3 constitute second components for the purposes of the proposed method and are thus divided into different groups on the basis of their depth T. In particular, delivery tubes 3 are produced with different depths T in order to provide the necessary groups of delivery tubes 3. Preferably, the groups differ from one another in their depth T by more than the manufacturing tolerance.

It is proposed that a first component, i.e. a shaped seal 5, of a specific batch be combined or put together with only one second component, i.e. a delivery tube 3, of a group which fits or matches to the specific batch. The group matching the specific batch is selected as a function of or depending on the at least one decisive significant magnitude of this batch, i.e. in particular as a function of or depending on the mean value and the standard deviation of the volume and compressibility of the shaped seals 5 in this batch, such that the essential mamagnitude, i.e. in particular the depth T of the recess 6, of the particular group leads to a desired target value, in this case the desired fill level, or a specific seal in the device 1. The selection is made in particular taking account of the error propagation and the available groups.

In the embodiment shown, the device 1 has further components, namely the piston 4 and the support ring 7, the magnitudes or the dimensions of which are crucial for achieving the desired value, i.e. the desired fill level, of the respective device 1. Accordingly, the significant magnitudes of the further components, particularly the diameter of the pistons 4 in a batch of pistons 4 and the width B of the recess 6, more precisely the magnitudes of the support ring 7 and delivery tube 3 which are crucial thereto, are preferably determined by taking random samples and from these further significant magnitudes, particularly the mean value and the standard deviation. These further significant magnitudes are preferably also taken into account in the above-mentioned selection of the group of delivery tubes 3, so as to achieve the desired target value, i.e. the desired fill level, and hence the desired seal and metering accuracy.

The magnitudes specified, such as the volume, compressibility, depth, width or the like, are merely possible significant magnitudes stated by way of example. Depending on the construction and design of the device 1, the manufacture of the components and especially the tolerances of the components, further and/or different magnitudes may be used as significant and/or essential magnitudes. Alternatively or additionally, instead of the fill level, other magnitudes may be used as the target value. Instead of the delivery tube 3, other components may also be combined, as "variable" components - i.e. components with different essential magnitudes divided into groups - with batches of other components in order to achieve a target value or improved metering accuracy in the finished device 1.

In order to deliver the liquid 2 or medicament, the first and second components, i.e. in particular the delivery tube 3 and the shaped seal 5, are moved relative to one another, the proposed combination of the components leading to an optimum seal between the components and hence to improved metering accuracy during delivery or application.

In the embodiment shown, the proposed device 1 is constructed in particular as an atomiser or inhaler. The liquid 2 is sucked into the pump chamber 9 by the piston 4 as it moves axially back and forth alternately through the feed channel 11 or is put under pressure therein and expelled through the nozzle 12, and delivered or administered, preferably atomised, i.e. a spray mist or aerosol A is formed from the liquid 2, as indicated in the Figure.

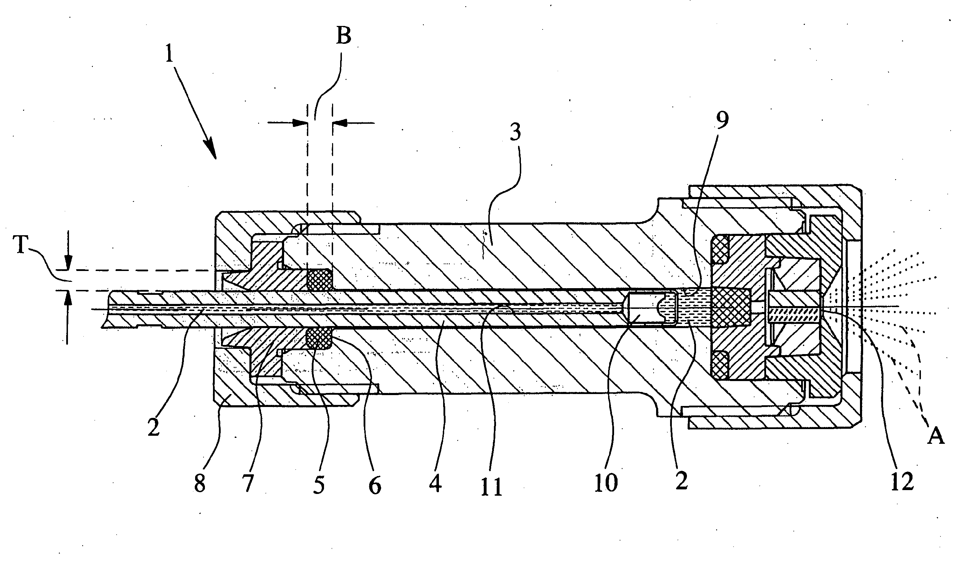

Particularly preferably, the device 1 is constructed as an atomiser or inhaler, as shown in principle in WO 91/14468 A1 and in a specific embodiment in WO 97/12687 A1 (Fig. 6a, 6b) and in Fig. 1 and 2 of WO 2005/080001 A1. Most preferably, it is the atomiser or inhaler sold by Boehringer Ingelheim GmbH under the brand name "Respimat".

However, the device I may for example also be used as a metering pump, particularly for accurately supplying medicaments or the like, especially as described in EP 1 426 662 A1.

In particular, the device 1 is a medical device. The liquid 2 is preferably a pharmaceutical composition, as already explained hereinbefore, or a medicament, therapeutic agent, diagnostic agent or the like.

The device 1 may also be used, in particular, to provide or deliver a number of active substances or medicaments simultaneously. In this case, the liquid 2 is, in particular, a suslution. The principle of a suslution is based on formulating a number of active substances alongside one another in one formulation in the form of a solution and a suspension. In this respect, reference is made to EP 1 087 750 A1.

However, the device I may theoretically also be used for cosmetic or other purposes.

Preferred ingredients and/or formulations of the medicament or liquid 2 are listed hereinafter:
The below mentioned compounds may be used on their own or combined with other active substances for use in the device according to this invention. These include, in particular, betamimetics, anticholinergics, corticosteroids, PDE4-inhibitors, LTD4-antagonists, EGFR-inhibitors, dopamin-agonists, antiallergic agents, PAF-antagonists und PI3-kinase inhibitors, but also combinations of two or three active substances, i.e:
   - Betamimetics with corticosteroids, PDE4-inhibitors, EGFR-inhibitors or LTD4-antagonists,
   - Anticholinergics with betamimetics, corticosteroids, PDE4-inhibitors, EGFR-inhibitors or LTD4-antagonists,
   - Corticosteroids with PDE4-inhibitors, EGFR-inhibitors or LTD4-antagonists
   - PDE4-inhibitors with EGFR-inhibtors or LTD4-antagonists
   - EGFR-inhibtors with LTD4-antagonists.
Examples of preferred betamimetics which may be mentioned include Albuterole, Arformoterole, Bambuterole, Bitolterole, Broxaterole, Carbuterole, Clenbuterole, Fenoterole, Formoterole, Hexoprenaline, Ibuterole, Isoetharine, Isoprenaline, Levosalbutamole, Mabuterole, Meluadrine, Metaproterenole, Orciprenaline, Pirbuterole, Procaterole, Reproterole, Rimiterole, Ritodrine, Salmefamole, Salmeterole, Soterenole, Sulphonterole, Terbutaline, Tiaramide, Tolubuterole, Zinterole, CHF-1035, HOKU-81, KUL-1248 and
   - 3-(4-{6-[2-Hydroxy-2-(4-hydroxy-3-hydroxymethyl-phenyl)-ethylamino]-hexyloxy}-butyl)-benzyl-sulfonamide
   - 5-[2-(5,6-Diethyl-indan-2-ylamino)-1-hydroxy-ethyl]-8-hydroxy-1H-quinolin-2-one
   - 4-Hydroxy-7-[2-{[2-([3-(2-phenylethoxy)propyl]sulphonyl}ethyl]-amino}ethyl]-2(3H)-benzothiazolone
   - 1-(2-Fluoro-4-hydroxyphenyl)-2-[4-(1-benzimidazolyl)-2-methyl-2-butylamino]ethanole
   - 1-[3-(4-Methoxybenzyl-amino)-4-hydroxyphenyl]-2-[4-(1-benzimidazolyl)-2-methyl-2-butylamino]ethanole
   - 1-[2H-5-hydroxy-3-oxo-4H-1,4-benzoxazin-8-yl]-2-[3-(4-N,N-dimethylaminophenyl)-2-methyl-2-propylamino]ethanole
   - 1-[2H-5-hydroxy-3-oxo-4H-1,4-benzoxazin-8-yl]-2-[3-(4-methoxyphenyl)-2-methyl-2-propylamino]ethanole
   - 1-[2H-5-hydroxy-3-oxo-4H-1,4-benzoxazin-8-yl]-2-[3-(4-n-butyloxyphenyl)-2-methyl-2-propylamino]ethanole
   - 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol
   - 5-Hydroxy-8-(1-hydroxy-2-isopropylaminobutyl)-2H-1,4-benzoxazin-3-(4H)-one
   - 1-(4-Amino-3-chloro-5-trifluormethylphenyl)-2-tert.-butylamino)ethanol
   - 6-Hydroxy-8-{1-hydroxy-2-[2-(4-methoxy-phenyl)-1,1-dimethyl-ethylamino]-ethyl}-4H-benzo [1,4]oxazin-3-one
   - 6-Hydroxy-8-{1-hydroxy-2-[2-(4-phenoxy-acetic acid ethylester)-1,1-dimethyl-ethylamino]-ethyl}-4H-benzo[1,4]oxazin-3-one
   - 6-Hydroxy-8-{1-hydroxy-2-[2-(4-phenoxy-acetic acid)-1,1-dimethyl-ethylamino]-ethyl}-4H-benzo[1,4]oxazin-3-one
   - 8-{2-[1,1-Dimethyl-2-(2,4,6-trimethylphenyl)-ethylamino]-1-hydroxyethyl}-6-hydroxy-4H-benzo[1,4]oxazin-3-one
   - 6-Hydroxy-8-{1-hydroxy-2-[2-(4-hydroxy-phenyl)-1,1-dimethyl-ethylamino]-ethyl}-4H-benzo[1,4]oxazin-3-one
   - 6-Hydroxy-8-{1-hydroxy-2-[2-(4-isopropyl-phenyl)-1,1dimethyl-ethylamino] -ethyl}-4H-benzo[1,4]oxazin-3-one
   - 8-{2-[2-(4-Ethyl-phenyl)-1,1-dimethyl-ethylamino]-1-hydroxy-ethyl}-6-hydroxy-4H-benzo[1,4]oxazin-3-one
   - 8-{2-[2-(4-Ethoxy-phenyl)-1,1-dimethyl-ethylamino]-1-hydroxy-ethyl}-6-hydroxy-4H-benzo[1,4]oxazin-3-one
   - 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-butyric acid
   - 8-{2-[2-(3,4-Difluor-phenyl)-1,1-dimethyl-ethylamino]-1-hydroxy-ethyl}-6-hydroxy-4H-benzo[1,4]oxazin-3-on
   - 1-(4-Ethoxy-carbonylamino-3-cyano-5-fluorophenyl)-2-(tert.-butylamino)ethanol
   - 2-Hydroxy-5-(1-hydroxy-2-{2-[4-(2-hydroxy-2-phenyl-ethylamino)-phenyl]-ethylamino}-ethyl)-benzaldehyde
   - N-[2-Hydroxy-5-(1-hydroxy-2-{2-[4-(2-hydroxy-2-phenyl-ethylamino)-phenyl]-ethylamino}-ethyl)-phenyl]-formamide
   - 8-Hydroxy-5-(1-hydroxy-2-{2-[4-(6-methoxy-biphenyl-3-ylamino)-phenyl]-ethylamino}-ethyl)-1H-quinolin-2-one
   - 8-Hydroxy-5-[1-hydroxy-2-(6-phenethylamino-hexylamino)-ethyl]-1H-quinolin-2-one
   - 5-(2-(2-{4-[4-(2-Amino-2-methyl-propoxy)-phenylamino]-phenyl}-ethylamino)-1-hydroxy-ethyl]-8-hydroxy-1H-quinolin-2-one
   - [3-(4-{6-[2-Hydroxy-2-(4-hydroxy-3-hydroxymethyl-phenyl)-ethylamino]-hexyloxy}-butyl)-5-methyl-phenyl]-urea
   - 4-(2-{6-[2-(2,6-Dichloro-benzyloxy)-ethoxy]-hexylamino}-1-hydroxyethyl)-2-hydroxymethyl-phenol
   - 3-(4-{6-[2-Hydroxy-2-(4-hydroxy-3-hydroxymethyl-phenyl)-ethylamino]-hexyloxy}-butyl)-benzenesulfonamide
   - 3-(3-{7-[2-Hydroxy-2-(4-hydroxy-3-hydroxymethyl-phenyl)-ethylamino]-heptyloxy)-propyl)-benzenesulfonamide
   - 4-(2-{6-[4-(3-Cyclopentanesulfonyl-phenyl)-butoxy]-hexylamino}-1-hydroxy-ethyl)-2-hydroxymethyl-phenol
   - N-Adamantan-2-yl-2-(3-{2-[2-hydroxy-2-(4-hydroxy-3-hydroxymethylphenyl)-ethylamino]-propyl}-phenyl)-acetamide
optionally in racemic form, as enantiomers, diastereomeres or as pharmacologically acceptable salts, solvates or hydrates. Preferred are salts selected from the group consisting of hydrochloride, hydrobromide, hydroiodide, hydrosulfate, hydrophosphate, hydromethansulfonate, hydronitrate, hydromaleate, hydroacetate, hydrocitrate, hydrofumarate, hydrotartrate, hydrooxalate, hydrosuccinate, hydrobenzoate und hydro-p-toluenesulfonate.

Examples of preferred anticholinergics which may be mentioned include Tiotropium salts, preferred the bromide salt, Oxitropium salts, preferred the bromide salt, Flutropium salts, preferred the bromide salt, Ipratropium salts, preferred the bromide salt, Glycopyrronium salts, preferred the bromide salt, Trospium salts, preferred the chloride salt, Tolterodin. From the above mentioned salts the pharmacologically active part is the cation, possible anions are chloride, bromide, iodide, sulfate, phosphate, methansulfonate, nitrate, maleate, acetate, citrate, fumarate, tartrate, oxalate, succinate, benzoate oder p-toluenesulfonate. Furthermore
- 2,2-Diphenylpropion acid tropenolester-methobromide
- 2,2-Diphenylpropion acid scopinester-methobromide
- 2-Fluor-2,2-Diphenylacetic acid scopinester-methobromide
- 2-Fluor-2,2-Diphenylacetic acid tropenolester-methobromide
- 3,3',4,4'-Tetrafluorbenzil acid tropenolester-Methobromide
- 3,3',4,4'-Tetrafluorbenzil acid scopinester-Methobromide
- 4,4'-Difluorbenzil acid tropenolester-Methobromide
- 4,4'-Difluorbenzil acid scopinester-Methobromide
- 3,3'-Difluorbenzil acid tropenolester-Methobromide
- 3,3'-Difluorbenzil acid scopinester-Methobromide
- 9-Hydroxy-fluoren-9-carbon acid tropenolester -Methobromide
- 9-Fluor-fluoren-9-carbon acid tropenolester -Methobromide
- 9-Hydroxy-fluoren-9-carbon acid scopinester -Methobromide
- 9-Fluor-fluoren-9-carbon acid scopinester Methobromide
- 9-Methyl-fluoren-9-carbon acid tropenolesterMethobromide
- 9-Methyl-fluoren-9-carbon acid scopinesterMethobromide
- Benzil acid cyclopropyltropinester-Methobromide
- 2,2-Diphenylpropion acid cyclopropyltropinester-Methobromide
- 9-Hydroxy-xanthen-9-carbon acid cyclopropyltropinesterMethobromide
- 9-Methyl-fluoren-9-carbon acid cyclopropyltropinester-Methobromide
- 9-Methyl-xanthen-9-carbon acid cyclopropyltropinester-Methobromide
- 9-Hydroxy-fluoren-9-carbon acid cyclopropyltropinester-Methobromide
- 4,4'-Difluorbenzil acid methylestercyclopropyltropinester-Methobromide
- 9-Hydroxy-xanthen-9-carbon acid tropenolester -Methobromide
- 9-Hydroxy-xanthen-9-carbon acid scopinester Methobromide
- 9-Methyl-xanthen-9-carbon acid tropenolester -Methobromide
- 9-Methyl-xanthen-9-carbon acid scopinesterMethobromide
- 9-Ethyl-xanthen-9-carbon acid tropenolester Methobromide
- 9-Difluormethyl-xanthen-9-carbon acid tropenolester -Methobromide
- 9-Hydroxymethyl-xanthen-9-carbon acid scopinester -Methobromide

Examples of preferred corticosteroids which may be mentioned include Beclomethasone, Betamethasone, Budesonide, Butixocorte, Ciclesonide, Deflazacorte, Dexamethasone, Etiprednole, Flunisolide, Fluticasone, Loteprednole, Mometasone, Prednisolone, Prednisone, Rofleponide, Triamcinolone, RPR-106541, NS-126, ST-26 and
- 6,9-Difluoro-17-[(2-furanylcarbonyl)oxy]-11-hydroxy-16-methyl-3-oxo-androsta-1,4-dien-17-carbothion acid (S)-fluoromethylester
- 6,9-Difluoro-11-hydroxy-16-methyl-3-oxo-17-propionyloxy-androsta-1,4-dien-17-carbothion acid (S)-(2-oxo-tetrahydro-furan-3S-yl)ester,
- 6a,9a-difluoro-11β-hydroxy-16α-methyl-3-oxo-17α-(2,2,3,3-tertamethylcyclopropylcarbonyl)oxy-androsta-1,4-diene-17β-carboxylic acid cyanomethyl ester
optionally in racemic form, as enantiomers, diastereomeres or as pharmacologically acceptable salts, solvates or hydrates. Examples for preferred salts and derivatives are alkali salts, i.e. sodium or potassium salts, sulfobenzoates, phosphates, isonicotinates, acetates, dichloroacetates, propionates, dihydrogenphosphates, palmitates, pivalates or furoates.

Examples of preferred PDE4-inhibtors which may be mentioned include Enprofylline, Theophylline, Roflumilaste, Ariflo (Cilomilast), Tofimilaste, Pumafentrine, Lirimilaste, Arofylline, Atizorame, D-4418, Bay-198004, BY343, CP-325,366, D-4396 (Sch-351591), AWD-12-281 (GW-842470), NCS-613; CDP-840, D-4418, PD-168787, T-440, T-2585, V-11294A, Cl-1018, CDC-801, CDC-3052, D-22888, YM-58997, Z-15370 and
- N-(3,5-Dichloro-1-oxo-pyridin-4-yl)-4-difluoromethoxy-3-cyclopropylmethoxybenzamide
- (-)p-[(4*a*R*,10*b*S*)-9-Ethoxy-1,2,3,4,4a,10b-hexahydro-8-methoxy-2-methylbenzo[s][1,6]naphthyridin-6-yl]-N,N-diisopropylbenzamid
- (R)-(+)-1-(4-Bromobenzyl)-4-[(3-cyclopentyloxy)-4-methoxyphenyl]-2-pyrrolidon
- 3-(Cyclopentyloxy-4-methoxyphenyl)-1-(4-N'-[N-2-cyano-S-methylisothioureido]benzyl)-2-pyrrolidone
- cis[4-Cyano-4-(3-cyclopentyloxy-4-methoxyphenyl)cyclohexan-1-carbon acid]
- 2-carbomethoxy-4-cyano-4-(3-cyclopropylmethoxy-4-difluoromethoxyphenyl)cyclohexan-1-one
- cis[4-Cyano-4-(3-cyclopropylmethoxy-4-difluoromethoxyphenyl)cyclohexan-1-ol]
- (R)-(+)-Ethyl[4-(3-cyclopentyloxy-4-methoxyphenyl)pyrrolidin-2-yliden]acetate
- (S)-(-)-Ethyl[4-(3-cyclopentyloxy-4-methoxyphenyl)pyrrolidin-2-yliden]acetate
- 9-Cyclopentyl-5,6-dihydro-7-ethyl-3-(2-thienyl)-9*H*-pyrazolo[3,4-c]-1,2,4-triazolo[4,3-a]pyridine
- 9-Cyclopentyl-5,6-dihydro-7-ethyl-3-(*tert*-butyl)-9*H*-pyrazolo[3,4-c]-1,2,4-triazolo[4,3-a]pyridine
optionally in racemic form, as enantiomers, diastereomeres or as pharmacologically acceptable salts, solvates or hydrates. Preferred are salts selected from the group consisting of hydrochloride, hydrobromide, hydroiodide, hydrosulfate, hydrophosphate, hydromethansulfonate, hydronitrate, hydromaleate, hydroacetate, hydrocitrate, hydrofumarate, hydrotartrate, hydrooxalate, hydrosuccinate, hydrobenzoate und hydro-p-toluenesulfonate.

Examples of preferred LTD4-antagonists which may be mentioned include Montelukaste, Pranlukaste, Zafirlukaste, MCC-847 (ZD-3523), MN-001, MEN-91507 (LM-1507), VUF-5078, VUF-K-8707, L-733321 and
- 1-(((R)-(3-(2-(6,7-Difluoro-2-quinolinyl)ethenyl)phenyl)-3-(2-(2- hydroxy-2-propyl)phenyl)thio)methylcyclopropane-acetic acid,
- 1-(((1(R)-3(3-(2-(2,3-Dichlorothieno[3,2-b]pyfidin-5-yl)-(E)-ethenyl)phenyl)-3-(2-(1-hydroxy-1-methytethyl)phenyl)-propyl)thio)methyl)cyclopropane acetic acid
- [2-[[2-(4-tert-Butyl-2-thiazolyl)-5-benzofuranyl]oxymethyl]phenyl]acetic acid
optionally in racemic form, as enantiomers, diastereomeres or as pharmacologically acceptable salts, solvates or hydrates. Preferred are salts selected from the group consisting of hydrochloride, hydrobromide, hydroiodide, hydrosulfate, hydrophosphate, hydromethansulfonate, hydronitrate, hydromaleate, hydroacetate, hydrocitrate, hydrofumarate, hydrotartrate, hydrooxalate, hydrosuccinate, hydrobenzoate und hydro-p-toluenesulfonate. Further examples for optionally preferred salts and derivatives are alkali salts, i.e. sodium or potassium salts, sulfobenzoates, dium or potassium salts, sulfobenzoates, phosphates, isonicotinates, acetates, propionates, dihydrogenphosphates, palmitates, pivalates or furoates.

Examples of preferred EGFR-inhibtors which may be mentioned include Cetuximabe, Trastuzumabe, ABX-EGF, Mab ICR-62 and
- 4-[(3-Chlor-4-fluorphenyl)amino]-6-{[4-(morpholin-4-yl)-1-oxo-2-buten-1-yl]amino}-7-cyclopropylmethoxy-chinazoline
- 4-[(3-Chlor-4-fluorphenyl)amino]-6-{[4-(N,N-diethylamino)-1-oxo-2-buten-1-yl]amino}-7-cyclopropylmethoxy-chinazoline
- 4-[(3-Chlor-4-fluorphenyl)amino]-6-{[4-(N,N-dimethylamino)-1-oxo-2-buten-1-yl]amino}-7-cyclopropylmethoxy-chinazoline
- 4-[(R)-(1-Phenyl-ethyl)amino]-6-{[4-(morpholin-4-yl)-1-oxo-2-buten-1-yl]amino}-7-cyclopentyloxy-chinazoline
- 4-[(3-Chlor-4-fluor-phenyl)amino]-6-{[4-((R)-6-methyl-2-oxo-morpholin-4-yl)-1-oxo-2-buten-1-yl]amino}-7-cyclopropylmethoxy-chinazoline
- 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazoline
- 4-[(3-Chlor-4-fluor-phenyl)amino]-6-{[4-((R)-2-methoxymethyl-6-oxo-morpholin-4-yl)-1-oxo-2-buten-1-yl]amino}-7-cyclopropylmethoxy-chinazoline
- 4-[(3-Chlor-4-fluor-phenyl)amino]-6-[2-((S)-6-methyl-2-oxo-morpholin-4-yl)-ethoxy]-7-methoxy-chinazoline
- 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazoline
- 4-[(3-Chlor-4-fluorphenyl)amino]-6-{[4-(N,N-dimethylamino)-1-oxo-2-buten-1-yl]amino}-7-cyclopentyloxy-chinazoline
- 4-[(R)-(1-Phenyl-ethyl)amino]-6-{[4-(N,N-bis-(2-methoxy-ethyl)-amino)-1-oxo-2-buten-1-yl]amino}-7-cydopropylmethoxy-chinazoline
- 4-[(R)-(1-Phenyl-ethyl)amino]-6-({4-[N-(2-methoxy-ethyl)-N-ethyl-amino]-1-oxo-2-buten-1-y1}amino)-7-cyclopropylmethoxy-chinazoline
- 4-[(R)-(1-Phenyl-ethyl)amino]-6-((4-[N-(2-methoxy-ethyl)-N-methyl-amino]-1-oxo-2-buten-1-yl}amino)-7-cyclopropylmethoxy-chinazoline
- 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazoline
- 4-[(3-Chlor-4-fluorphenyl)amino]-6-{[4-(N,N-dimethylamino)-1-oxo-2-buten-1-yl]amino}-7-((R)-tetrahydrofuran-3-yloxy)-chinazoline
- 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazoline
- 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazoline
- 4-[(3-Chlor-4-fluorphenyl)amino]-6-{[4-(N-cyclopropyl-N-methyl-amino)-1-oxo-2-buten-1-yl]amino}-7-cyclopentyloxy-chinazoline
- 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazoline
- 4-[(3-Chlor-4-fluorphenyl)amino]-6-{[4-(N,N-dimethylamino)-1-oxo-2-buten-1-yl]amino}-7-[(S)-(tetrahydrofuran-2-yl)methoxy]-chinazoline
- 4-[(3-Ethinyl-phenyl)amino]-6,7-bis-(2-methoxy-ethoxy)-chinazoline
- 4-[(3-Chlor-4-fluorphenyl)amino]-7-[3-(morpholin-4-yl)-propyloxy]-6-[(vinylcarbonyl)amino]-chinazoline
- 4-[(R)-(1-Phenyl-ethyl)amino]-6-(4-hydroxy-phenyl)-7H-pyrrolo[2,3-d]pyrimidine
- 3-Cyano-4-[(3-chlor-4-fluorphenyl)amino]-6-{[4-(N,N-dimethylamino)-1-oxo-2-buten-1-yl]amino}-7-ethoxy-chinoline
- 4-{[3-Chlor-4-(3-fluor-benzyloxy)-phenyl]amino}-6-(5-{[(2-methansulfonyl-ethyl)amino]methyl}-furan-2-yl)chinazoline
- 4-[(R)-(1-Phenyl-ethyl)amino]-6-{[4-((R)-6-methyl-2-oxo-morpholin-4-yl)-1-oxo-2-buten-1-yl]amino}-7-methoxy-chinazoline
- 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazoline
- 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazoline
- 4-[(3-Ethinyl-phenyl)amino]-6-{[4-(5,5-dimethyl-2-oxo-morpholin-4-yl)-1-oxo-2-buten-1-yl]amino}-chinazoline
- 4-[(3-Chlor-4-fluor-phenyl)amino]-6-[2-(2,2-dimethyl-6-oxo-morpholin-4-yl)-ethoxy]-7-methoxy-chinazoline
- 4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazoline
- 4-[(3-Chlor-4-fluor-phenyl)amino]-7-[2-(2,2-dimethyl-6-oxo-morpholin-4-yl)-ethoxy]-6-[(S)-(tetrahydrofuran-2-yl)methoxy]-chinazoline
- 4-[(3-Chlor-4-fluor-phenyl)amino]-6-{2-[4-(2-oxo-morpholin-4-yl)-piperidin-1-yl]-ethoxy}-7-methoxy-chinazoline
- 4-[(3-Chlor-4-fluor-phenyl)amino]-6-[1-(tert.-butyloxycarbonyl)-piperidin-4-yloxy]-7-methoxy-chinazoline
- 4-[(3-Chlor-4-fluor-phenyl)amino]-6-(trans-4-amino-cyclohexan-1-yloxy)-7-methoxy-chinazoline
- 4-[(3-Chlor-4-fluor-phenyl)amino]-6-(trans-4-methansulfonylamino-cyclohexan-1-yloxy)-7-methoxy-chinazoline
- 4-[(3-Chlor-4-fluor-phenyl)amino]-6-(tetrahydropyran-3-yloxy)-7-methoxy-chinazoline
- 4-[(3-Chlor-4-fluor-phenyl)amino]-6-(1-methyl-piperidin-4-yloxy)-7-methoxy-chinazoline
- 4-[(3-Chlor-4-fluor-phenyl)amino]-6-{1-[(morpholin-4-yl)carbonyl]-piperidin-4-yloxy}-7-methoxy-chinazoline
- 4-[(3-Chlor-4-fluor-phenyl)amino]-6-{1-[(methoxymethyl)carbonyl]-piperidin-4-yloxy}-7-methoxy-chinazoline
- 4-[(3-Chlor-4-fluor-phenyl)amino]-6-(piperidin-3-yloxy)-7-methoxy-chinazoline
- 4-[(3-Chlor-4-fluor-phenyl)amino]-6-[1-(2-acetylamino-ethyl)-piperidin-4-yloxy]-7-methoxy-chinazoline
- 4-[(3-Chlor-4-fluor-phenyl)amino]-6-(tetrahydropyran-4-yloxy)-7-ethoxy-chinazoline
- 4-[(3-Chlor-4-fluor-phenyl)amino]-6-((S)-tetrahydrofuran.3.yloxy)-7-hydroxy-chinazoline
- 4-[(3-Chlor-4-fluor-phenyl)amino]-6-(tetrahydropyran-4-yloxy)-7-(2-methoxy-ethoxy)-chinazoline
- 4-[(3-Chlor-4-fluor-phenyl)amino]-6-{trans-4-[(dimethylamino)sulfonylamino]-cyclohexan-1-yloxy}-7-methoxy-chinazoline
- 4-[(3-Chlor-4-fluor-phenyl)amino]-6-{trans-4-[(morpholin-4-yl)carbonylamino]-cyclohexan-1-yloxy}-7-methoxy-chinazoline
- 4-[(3-Chlor-4-fluor-phenyl)amino]-6-{trans-4-[(morpholin-4-yl)sulfonylamino]-cyclohexan-1-yloxy}-7-methoxy-chinazoline
- 4-[(3-Chlor-4-fluor-phenyl)amino]-6-(tetrahydropyran-4-yloxy)-7-(2-acetylamino-ethoxy)-chinazoline
- 4-[(3-Chlor-4-fluor-phenyl)amino]-6-(tetrahydropyran-4-yloxy)-7-(2-methansulfonylamino-ethoxy)-chinazoline
- 4-[(3-Chlor-4-fluor-phenyl)amino]-6-{1-[(piperidin-1-yl)carbonyl]-piperidin-4-yloxy}-7-methoxy-chinazoline
- 4-[(3-Chlor-4-fluor-phenyl)amino]-6-(1-aminocarbonylmethyl-piperidin-4-yloxy)-7-methoxy-chinazoline
- 4-[(3-Chlor-4-fluor-phenyl)amino]-6-(cis-4-{N-[(tetrahydropyran-4-yl)carbonyl]-N-methyl-amino}-cyclohexan-1-yloxy)-7-methoxy-chinazoline
- 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazoline
- 4-[(3-Chlor-4-fluor-phenyl)amino]-6-(cis-4-{N-[(morpholin-4-yl)sulfonyl]-N-methyl-amino}-cyclohexan-1-yloxy)-7-methoxy-chinazoline
- 4-[(3-Chlor-4-fluor-phenyl)amino]-6-(trans-4-ethansulfonylamino-cyclohexan-1-yloxy)-7-methoxy-chinazoline
- 4-[(3-Chlor-4-fluor-phenyl)amino]-6-(1-methansulfonyl-piperidin-4-yloxy)-7-ethoxy-chinazoline
- 4-[(3-Chlor-4-fluor-phenyl)amino]-6-(1-methansulfonyl-piperidin-4-yloxy)-7-(2-methoxy-ethoxy)-chinazoline
- 4-[(3-Chlor-4-fluor-phenyl)amino]-6-[1-(2-methoxy-acetyl)-piperidin-4-yloxy]-7-(2-methoxy-ethoxy)-chinazoline
- 4-[(3-Chlor-4-fluor-phenyl)amino]-6-(cis-4-acetylamino-cyclohexan-1-yloxy)-7-methoxy-chinazoline
- 4-[(3-Ethinyl-phenyl)amino]-6-[1-(tert.-butyloxycarbonyl)-pipefidin-4-yloxy]-7-methoxy-chinazoline
- 4-[(3-Ethinyl-phenyl)amino]-6-(tetrahydropyran-4-yloxy]-7-methoxy-chinazoline
- 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazoline
- 4-[(3-Chlor-4-fluor-phenyl)amino]-6-(cis-4-{N-[(4-methyl-piperazin-1-yl)carbonyl]-N-methyl-amino}-cyclohexan-1-yloxy)-7-methoxy-chinazoline
- 4-[(3-Chlor-4-fluor-phenyl)amino]-6-{cis-4-[(morpholin-4-yl)carbonylamino]-cyclohexan-1-yloxy}-7-methoxy-chinazoline
- 4-[(3-Chlor-4-fluor-phenyl)amino]-6-{1-[2-(2-oxopyrrolidin-l-yl)ethyl]-piperidin-4-yloxy}-7-methoxy-chinazoline
- 4-[(3-Chlor-4-fluor-phenyl)amino]-6-{1-[(morpholin-4-yl)carbonyl]-piperidin-4-yloxy}-7-(2-methoxy-ethoxy)-chinazoline
- 4-[(3-Ethinyl-phenyl)amino]-6-(1-acetyl-piperidin-4-yloxy)-7-methoxy-chinazoline
- 4-[(3-Ethinyl-phenyl)amino]-6-(1-methyl-piperidin-4-yloxy)-7-methoxy-chinazoline
- 4-[(3-Ethinyl-phenyl)amino]-6-(1-methansulfonyl-piperidin-4-yloxy)-7-methoxy-chinazoline
- 4-[(3-Chlor-4-fluor-phenyl)amino]-6-(1-methyl-pipefidin-4-yloxy)-7(2-methoxy-ethoxy)-chinazoline
- 4-[(3-Chlor-4-fluor-phenyl)amino]-6-(1-isopropyloxycarbonyl-piperidin-4-yloxy)-7-methoxy-chinazoline
- 4-[(3-Chlor-4-fluor-phenyl)amino]-6-(cis-4-methylamino-cyclohexan-1-yloxy)-7-methoxy-chinazoline
- 4-[(3-Chlor-4-fluor-phenyl)amino]-6-{cis-4-[N-(2-methoxy-acetyl)-N-methyl-amino]-cyclohexan-1-yloxy}-7-methoxy-chinazoline
- 4-[(3-Ethinyl-phenyl)amino]-6-(pipefidin-4-yloxy)-7-methoxy-chinazoline
- 4-[(3-Ethinyl-phenyl)amino]-6-[1-(2-methoxy-acetyl)-piperidin-4-yloxy]-7-methoxy-chinazoline
- 4-[(3-Ethinyl-phenyl)amino]-6-{1-[(morpholin-4-yl)carbonyl]-piperidin-4-yloxy}-7-methoxy-chinazoline
- 4-[(3-Chlor-4-fluor-phenyl)amino]-6-{1-[(cis-2,6-dimethyl-morpholin-4-yl)carbonyl]-piperidin-4-yloxy}-7-methoxy-chinazoline
- 4-[(3-Chlor-4-fluor-phenyl)amino]-6-{1-[(2-methyl-morpholin-4-yl)carbonyl]-piperidin-4-yloxy}-7-methoxy-chinazoline
- 4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazoline
- 4-[(3-Chlor-4-fluor-phenyl)amino]-6-{1-[(N-methyl-N-2-methoxyethyl-amino)carbonyl]-piperidin-4-yloxy}-7-methoxy-chinazoline
- 4-[(3-Chlor-4-fluor-phenyl)amino]-6-(1-ethyl-piperidin-4-yloxy)-7-methoxy-chinazoline
- 4-[(3-Chlor-4-fluor-phenyl)amino]-6-{1-[(2-methoxyethyl)carbonyl]-piperidin-4-yloxy}-7-methoxy-chinazoline
- 4-[(3-Chlor-4-fluor-phenyl)amino]-6-{1-[(3-methoxypropyl-amino)-carbonyl]-piperidin-4-yloxy}-7-methoxy-chinazoline
- 4-[(3-Chlor-4-fluor-phenyl)amino]-6-[cis-4-(N-methansulfonyl-N-methyl-amino)-cyclohexan-1-yloxy]-7-methoxy-chinazoline
- 4-[(3-Chlor-4-fluor-phenyl)amino]-6-[cis-4-(N-acetyl-N-methyl-amino)-cyclohexan-1-yloxy]-7-methoxy-chinazoline
- 4-[(3-Chlor-4-fluor-phenyl)amino]-6-(trans-4-methylamino-cyclohexan-1-yloxy)-7-methoxy-chinazoline
- 4-[(3-Chlor-4-fluor-phenyl)amino]-6-[trans-4-(N-methansulfonyl-N-methyl-amino)-cydohexan-1-yloxy]-7-methoxy-chinazoline
- 4-[(3-Chlor-4-fluor-phenyl)amino]-6-(trans-4-dimethylamino-cyclohexan-1-yloxy)-7-methoxy-chinazoline
- 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazoline
- 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazoline
- 4-[(3-Chlor-4-fluor-phenyl)amino]-6-(1-methansulfonyl-piperidin-4-yloxy)-7-methoxy-chinazoline
- 4-[(3-Chlor-4-fluor-phenyl)amino]-6-(1-cyano-piperidin-4-yloxy)-7-methoxy-chinazoline
optionally in racemic form, as enantiomers, diastereomeres or as pharmacologically acceptable salts, solvates or hydrates. Preferred are salts selected from the group consisting of hydrochloride, hydrobromide, hydroiodide, hydrosulfate, hydrophosphate, hydromethansulfonate, hydronitrate, hydromaleate, hydroacetate, hydrocitrate, hydrofumarate, hydrotartrate, hydrooxalate, hydrosuccinate, hydrobenzoate und hydro-p-toluenesulfonate.

Examples of preferred dopamin antagonists which may be mentioned include Bromocriptine, Cabergoline, Alpha-Dihydroergocryptine, Lisuride, Pergolide, Pramipexole, Roxindole, Ropinirole, Talipexole, Terguride and Viozane, optionally in racemic form, as enantiomers, diastereomeres or as pharmacologically acceptable salts, solvates or hydrates. Preferred are salts selected from the group consisting of hydrochloride, hydrobromide, hydroiodide, hydrosulfate, hydrophosphate, hydromethansulfonate, hydronitrate, hydromaleate, hydroacetate, hydrocitrate, hydrofumarate, hydrotartrate, hydrooxalate, hydrosuccinate, hydrobenzoate und hydro-p-toluenesulfonate.

Examples of preferred antiallergic agents which may be mentioned include Epinastine, Cetirizine, Azelastine, Fexofenadine, Levocabastine, Loratadine, Mizolastine, Ketotifene, Emedastine, Dimetindene, Clemastine, Bamipine, Cexchlorpheniramine, Pheniramine, Doxylamine, Chlorphenoxamine, Dimenhydrinate, Diphenhydramine, Promethazine, Ebastine, Desloratidine and Meclozine, optionally in racemic form, as enantiomers, diastereomeres or as pharmacologically acceptable salts, solvates or hydrates. Preferred are salts selected from the group consisting of hydrochloride, hydrobromide, hydroiodide, hydrosulfate, hydrophosphate, hydromethansulfonate, hydronitrate, hydromaleate, hydroacetate, hydrocitrate, hydrofumarate, hydrotartrate, -hydrooxalate, hydrosuccinate, hydrobenzoate und hydro-p-toluenesulfonate.

Moreover, inhalable macromolecules can be used as pharmacologically active substances, as disclosed in EP 1 003 478 A1 or CA 2297174 A1.

Moreover, the compound could be from the group of derivates of ergotalcaloids, triptane, CGRP-antagonists, phosphodiesterase-V-inhibitores, optionally in the form of the racemates, the enantiomers, the diastereomers and optionally the pharmacologically acceptable acid addition salts and the hydrates thereof.

As derivates of alkaloides: dihydroergotamine, ergotamine.

## Claims

1. Method of assembling a device (1) for delivering a medicament, particularly in the form of an aerosol (A), with improved metering accuracy,
wherein at least one first and one second component are provided, which are sealingly brought into engagement with one another or placed against one another,
wherein the first component is produced in batches,
wherein at least one significant magnitude of the first components in each batch is determined only by random sampling,
wherein at least one decisive significant magnitude for all the first components of the respective batch is determined from the at least one significant magnitude,
wherein the second component is divided into groups, which differ, by at least one essential magnitude of the second components,
wherein depending on the at least one decisive significant magnitude a group matching to the respective decisive significant magnitude and, thus, to the respective batch is selected,
wherein a first component of a batch is combined with a second component of a group matching to this batch, so as to achieve an optimum seal between these two components in order to improve the metering accuracy, and these two components are moved relative to one another for the delivery process.

2. Method according to claim 1, **characterized in that** the first components are injection moulded, and/or that the first components are shaped seals (5), preferably annular-like, particularly O-rings, and/or that a tool-related dimension, the volume and/or the compressibility of the first components is or are determined as significant magnitude or magnitudes.

3. Method according to one of the preceding claims, **characterized in that** the mean value and the standard deviation are detected or determined as significant magnitudes, and/or that the mean value and the standard deviation are used as essential magnitudes for dividing into the groups.

4. Method according to one of the preceding claims, **characterized in that** the second components each have a recess (6), particularly a shoulder or groove, for accommodating the first component and preferably form a guide tube for a piston (4), preferably wherein a tool-related dimension, particularly the depth and/or width, of the recess is used as essential magnitude for dividing into the groups.

5. Method according to one of the preceding claims, **characterized in that** the second components are produced with different essential magnitudes, and in particular wherein the difference between the essential magnitudes of different groups is greater than the manufacturing tolerance, and/or that the second components are produced in batches, the essential magnitude of the second components in each batch being determined only by random sampling and from this the essential magnitude for all the second components of the respective batch is determined for the division into the groups.

6. Method according to one of the preceding claims, **characterized in that** the device (1) comprises, in addition to the first and second components, at least one further component, particularly a piston (4), which is sealed by the first component, and/or a support ring (7) for axially securing the first component to the second component, preferably wherein a further component, particularly the piston (4), is produced in batches and at least one significant magnitude, such as the diameter, is determined only by random sampling of each batch, more preferably wherein at least one or more other significant magnitude(s), particularly tool-related dimensions, of the further component or components, particularly the diameter of the piston (4) and/or the axially effective length of the support ring (3), is or are determined and into consideration as further significant magnitude(s) in addition to the decisive significant magnitude when selecting the matching group.

7. Method according to one of the preceding claims, **characterized in that** the matching group is selected such that the decisive significant magnitude together with optionally at least one further significant magnitude of a further component and together with the essential magnitude(s) leads to a target value, particularly a desired fill level of a recess by a seal and/or gasket.

## Patentansprüche

1. Verfahren zum Aufbau einer Vorrichtung (1) zur Bereitstellung bzw. Ausgabe eines Arzneimittels, insbesondere in Form eines Aerosols (A), mit verbesserter Dosiergenauigkeit,
wobei mindestens eine erste und eine zweite Komponente bzw. ein erster und ein zweiter Bauteil bereitgestellt werden, die dicht miteinander in Eingriff gebracht oder gegeneinander angeordnet werden,
wobei die erste Komponente in Chargen hergestellt wird,
wobei mindestens eine signifikante Größe der ersten Komponente in jeder Charge nur stichprobenartig erfasst wird,
wobei daraus mindestens eine entscheidende signifikante Größe für alle ersten Komponenten der jeweiligen Charge aus der mindestens einen signifikanten Größe bestimmt wird,
wobei die zweite Komponente in Gruppen aufgeteilt wird, die sich durch mindestens eine wesentliche Größe der zweiten Komponenten unterscheiden,
wobei in Abhängigkeit von der mindestens einen entscheidenden signifikanten Größe eine zu der jeweils entscheidenden signifikanten Größe und somit zur jeweiligen Charge passende Gruppe ausgewählt wird,
wobei eine erste Komponente einer Charge mit einer zweiten Komponente einer zu dieser Charge passenden Gruppe kombiniert wird, um eine optimale Abdichtung zwischen diesen beiden Komponenten zu erreichen, um die Dosiergenauigkeit zu verbessern, und diese beiden Komponenten werden zum Bereitstellungsvorgang relativ zueinander bewegt.

2. Verfahren nach Anspruch 1, **dadurch gekennzeichnet, dass** die ersten Komponenten spritzgegossen werden und/oder dass die ersten Komponenten Formdichtungen (5) darstellen, vorzugsweise ringförmig, insbesondere O-Ringe, und/oder dass ein Werkzeug bezogenes Maß, das Volumen und/oder die Kompressibilität der ersten Komponenten, als signifikante Größe(n) erfasst bzw. bestimmt wird bzw. werden.

3. Verfahren nach einem der vorhergehenden Ansprüche, **dadurch gekennzeichnet, dass** der Mittelwert und die Standardabweichuvg als signifikante Größen gemessen oder bestimmt werden, und/oder dass der Mittelwert und die Standardabweichung als wesentliche Größen zur Einteilung in die Gruppen verwendet werden.

4. Verfahren nach einem der vorhergehenden Ansprüche, **dadurch gekennzeichnet, dass** die zweiten Komponenten jeweils eine Ausnehmung (6), insbesondere eine Schulter oder Nut, zur Aufnahme der ersten Komponente aufweisen und vorzugsweise ein Führungsrohr für einen Kolben (4) bilden, wobei ein Werkzeug bezogenes Maß, insbesondere die Tiefe und/oder Breite, der Ausnehmung als wesentliche Größe zur Einteilung in die Gruppen verwendet wird.

5. Verfahren nach einem der vorhergehenden Ansprüche, **dadurch gekennzeichnet, dass** die zweiten Komponenten mit unterschiedlichen wesentlichen Grössen hergestellt werden, und wobei insbesondere die Differenz zwischen wesentlichen Grössen unterschiedlicher Gruppen größer ist als die Herstellungstoleranz, und/oder dass die zweiten Komponenten in Chargen hergestellt werden, wobei die wesentliche Größe der zweiten Komponenten in jeder Charge nur stichprobenartig erfasst und daraus die wesentliche Größe für alle zweiten Komponenten der jeweiligen Charge zur Einteilung in die Gruppen bestimmt wird.

6. Verfahren nach einem der vorhergehenden Ansprüche, **dadurch gekennzeichnet, dass** die Vorrichtung (1) zusätzlich zu der ersten und zweiten Komponente mindestens eine weitere Komponente aufweist, insbesondere einen Kolben (4), der durch die erste Komponente abgedichtet wird, und/oder einen Stütz- bzw. Haltering (7) zur axialen Befestigung bzw. Sicherung der ersten Komponente an der zweiten Komponente, wobei vorzugsweise eine weitere Komponente, insbesondere der Kolben (4), in Chargen hergestellt wird, und von jeder Charge nur stichprobenartig mindestens eine signifikante Größe, wie der Durchmesser, bestimmt wird, wobei bevorzugter mindestens eine oder mehrere weitere signifikante Größe(n), insbesondere Werkzeug bezogene Maße, der weiteren Komponente oder der weiteren Komponenten, insbesondere der Durchmesser des Kolbens (4) und/oder die axial effektive Länge des Stütz- bzw. Halterings (3) ist oder erfasst bzw. bestimmt und als weitere signifikante Größe(n) zusätzlich zu der massgeblichen signifikanten Grösse bei der Auswahl der passenden Gruppe berücksichtigt wird oder werden.

7. Verfahren nach einem der vorhergehenden Ansprüche, **dadurch gekennzeichnet, dass** die passende Gruppe derart ausgewählt wird, dass die entscheidende signifikante Grösse gegebenenfalls zusammen mit mindestens einer weiteren signifikanten Größe einer weiteren Komponente und zusammen mit der wesentlichen Größe(n) zu einem Zielwert bzw. Sollwert, insbesondere einem gewünschten Füllgrad einer Ausnehmung durch eine Dichtung und/oder eine Abdichtung, führt.

## Revendications

1. Méthode d'assemblage d'un dispositif (1) pour administrer un médicament, en particulier sous la forme d'un aérosol (A), avec une précision de dosage améliorée,
✔ dans laquelle sont prévus au moins un premier et un second composants, qui sont mis en prise de façon étanche l'un avec l'autre ou placés l'un contre l'autre,
✔ dans laquelle le premier composant est produit par lots,
✔ dans laquelle au moins une grandeur significative des premiers composants dans chaque lot est déterminée uniquement par échantillonnage aléatoire,
✔ dans laquelle au moins une grandeur significative décisive pour l'ensemble des premiers composants du lot respectif est déterminée à partir de ladite grandeur significative,
✔ dans laquelle le second composant est divisé en groupes, qui diffèrent, d'au moins une grandeur essentielle des seconds composants,
✔ dans laquelle en fonction de ladite grandeur significative décisive un groupe correspondant à la grandeur significative décisive respective et, ainsi, au lot respectif, est choisi,
✔ dans laquelle un premier composant d'un lot est combiné avec un second composant d'un groupe correspondant à ce lot, de sorte à assurer un joint optimal entre ces deux composants afin d'améliorer la précision de dosage, et ces deux composants sont déplacés l'un par rapport à l'autre pour le procédé d'administration.

2. Méthode selon la revendication 1, **caractérisée en ce que** les premiers composants sont moulés par injection, et/ou **en ce que** les premiers composants sont des joints profilés (5), de préférence de type annulaire, en particulier des joints toriques, et/ou **en ce qu'**une dimension relative à l'outil, le volume et/ou la compressibilité des premiers composants est ou sont déterminé(s) en tant que grandeur ou grandeurs significative(s).

3. Méthode selon l'une des revendications précédentes, **caractérisée en ce que** la valeur moyenne et l'écart standard sont détectés ou déterminés en tant que grandeurs significatives, et/ou **en ce que** la valeur moyenne et l'écart standard sont utilisés en tant que grandeurs essentielles pour assurer la division en groupes.

4. Méthode selon l'une des revendications précédentes, **caractérisée en ce que** les seconds composants présentent chacun un évidement (6), en particulier un épaulement ou une rainure, pour loger le premier composant et forment de préférence un tube de guidage pour un piston (4), de préférence dans laquelle une dimension relative à l'outil, en particulier la profondeur et/ou la largeur, de l'évidement est utilisée comme grandeur essentielle pour assurer la division en groupes.

5. Méthode selon l'une des revendications précédentes, **caractérisée en ce que** les seconds composants sont produits avec des grandeurs essentielles différentes, et en particulier dans laquelle la différence entre les grandeurs essentielles de groupes différents est supérieure à la tolérance de fabrication, et/ou **en ce que** les seconds composants sont produits par lots, la grandeur essentielle des seconds composants dans chaque lot étant déterminée uniquement par échantillonnage aléatoire, suite à quoi la grandeur essentielle pour l'ensemble des seconds composants du lot respectif est déterminée pour la division en groupes.

6. Méthode selon l'une des revendications précédentes, **caractérisée en ce que** le dispositif (1) comprend, en plus des premiers et seconds composants, au moins un autre composant, en particulier un piston (4), qui est scellé par le premier composant, et/ou un anneau de support (7) pour immobiliser axialement le premier composant sur le second composant, dans laquelle de préférence un autre composant, en particulier le piston (4), est produit par lots et au moins une grandeur significative, telle que le diamètre, est déterminée uniquement par échantillonnage aléatoire de chaque lot, dans laquelle plus préférablement au moins une ou plusieurs autre(s) grandeur(s) significative(s), en particulier les dimensions relatives à l'outil, de l'autre composant ou des autres composants, en particulier le diamètre du piston (4) et/ou la longueur axialement effective de l'anneau de support (3), est ou sont déterminée(s) et prise(s) en compte en tant qu'autre(s) grandeur(s) significative(s) en plus de la grandeur significative décisive lors du choix du groupe correspondant.

7. Méthode selon l'une des revendications précédentes, **caractérisée en ce que** le groupe correspondant est choisi de telle sorte que la grandeur significative décisive, éventuellement conjointement avec au moins une autre grandeur significative d'un autre composant et conjointement avec la (les) grandeur(s) essentielle(s), conduise à une valeur cible, en particulier à un niveau de remplissage souhaité d'un évidement par un joint et/ou une garniture.
